# EUROPEAN PATENT SPECIFICATION

(11) **EP 1 278 818 B1**
(45) Date of publication and mention of the grant of the patent: **22.02.2006**
(21) Application number: 01928339.9
(22) Date of filing: 30.03.2001
(51) Int. Cl.: A61L 12/08, A61K 9/00

(54) **USE OF LOW IONIC STRENGTH OPHTHALMIC COMPOSITIONS**
VERWENDUNG VON OPHTHALMISCHEN ZUSAMMENSETZUNGEN MIT NIEDRIGER IONENSTÄRKE
UTILISATION DES COMPOSITIONS OPHTHALMIQUES A FAIBLE FORCE IONIQUE

(30) Priority: 02.05.2000 US 563625
(43) Date of publication of application: 29.01.2003
(73) Proprietor: BAUSCH & LOMB INCORPORATED, Rochester, New York 14604-2701 (US)
(72) Inventor: HU, Zhenze, Pittsford, NY 14534 (US); SALAMONE, Joseph, C., Boca Raton, FL 33486 (US); HEILER, David, J., Avon, NY 14414 (US); SMERBECK, Richard, V., Pittsford, NY 14534 (US)
(74) Representative: Bowman, Paul Alan
(86) International application number: PCT/US2001/010274
(87) International publication number: WO 2001/082984

(56) References cited:
- EP-A- 0 923 950
- WO-A-00/37048
- WO-A-00/54747
- WO-A-01/74330
- WO-A-95/00618
- WO-A-97/43373
- WO-A-99/24543
- DE-A- 3 440 352
- US-A- 4 321 261

## Description

### BACKGROUND OF THE INVENTION

It has long been known in the art that a contact lens must have surfaces that have a certain degree of hydrophilicity in order to be wet by tears, thus providing unblurred vision.

Often hydrophilic monomers can be added to a mixture of comonomers in the formation of contact lenses so that upon polymerization optically clear contact lenses result that have a certain degree of hydrophilicity. As the hydrophilic monomer content increases where it is added directly to the lens composition, the physical characteristics of the lenses are affected by the increased hydration propensity of the polymeric composition.

In some cases, it has been known to treat a formed contact lens with a polymerizable hydrophilic monomer to form a surface coating of hydrophilic polymer grafted to an otherwise hydrophobic polymer surface. Although effective, this method of increasing the hydrophilic character of the lens surface can suffer from involved and difficult manufacturing procedures as well as lack of permanence.

Present rigid and soft contact lenses sometimes retain water on their surfaces through secondary chemical bonding and as a consequence only a very thin layer of water molecules is present between the eye and the contact lens.

Soft lenses are inherently comfortable but often times, as with hard lenses, suffer from brief surface dryness between eye blinks. State of the art technology teaches that a water soluble polymer may be applied to the surfaces of a hard contact lens to provide a "cushion" layer between the lens and the eye which is equated with increased wettability as well as wearer comfort and tolerance.

U.S. Patents 4,168,112, 4,321,261 and 4,436,730, all issued to Ellis et al., disclose methods for treating a charged contact lens surface with an oppositely charged ionic polymer to form a polyelectrolyte complex on the lens surface that improves wettability.

U.S. Patent 4,287,175 to Katz discloses a method of wetting a contact lens that comprises inserting a water-soluble solid polymer into the cul-de-sac of the eye. The disclosed polymers include cellulose derivatives, acrylates and natural products such as gelatin, pectins and starch derivatives.

U.S. Patent 5,397,848 to Yang et al. discloses a method of incorporating hydrophilic constituents into silicone polymer materials for use in contact and intraocular lenses.

U.S. Patents 5,700,559 and 5,807,636, both to Sheu et al., disclose hydrophilic articles (for example, contact lenses) comprising a substrate, an ionic polymeric layer on the substrate and a disordered polyelectrolyte coating ionically bonded to the polymeric layer.

U.S. Patent 5,705,583 to Bowers et al. discloses biocompatible polymeric surface coatings. The polymeric surface coatings disclosed include coatings synthesized from monomers bearing a center of positive charge, including cationic and zwitterionic monomers.

If the "cushion" layer dissipates rapidly, the wearer begins to feel discomfort between blinks and must rewet the lens surface. Thus it would be desirable to increase the useful residence time of a polymeric cushion layer formed by a wetting solution on a contact lens.

### SUMMARY OF THE INVENTION

It is an object of this invention to provide an improved method for wetting a hard or soft synthetic polymer contact lens by enhancing the durability of a thin layer of polyelectrolyte complex coating the lens surface and electrostatically bound thereto.

The invention provides, a method for wetting a contact lens having a surface comprising anionic sites which comprises administering an aqueous wetting solution comprising 0.001 to 10.0 percent by weight of a cationic cellulose polymer provided that the solution has an ionic strength as defined here of less than 0.10, preferably less than 0.095, and more preferably less than 0.090.

The layer or coating comprises a polyelectrolyte complex which is formed by complexation of an ionic lens surface with an oppositely charged ionic polymer, and this complex forms a hydrogel at the lens surface which absorbs water, has good water retention, and is compatible with the physiological structures of the eye. A durable "cushion" is formed which provides long lasting comfort to the eye. For a discussion of the measurement of polymer-surfactant interactions, see Argillier et al. "Polymer-Surfactant Interactions Studied with the Surface Force Apparatus" 146 Journal of Colloid and Interface Science 242 (1991).

In accordance with the present invention, it has been found that controlling the ionic strength of the wetting solution surprisingly improves the durability of the polymeric cushion formed on the surface of the contact lens.

The solutions of the invention are characterized herein using the term "ionic strength". The term "ionic strength" as used herein is a dimensionless number defined by the equation:
Ionic strength = 0.5Σ(CᵢZᵢ²), where Cᵢ is the molar concentration of ionic species i, and Zᵢ is the valence of ionic species i.

The ionic strength of the solution of the present invention is less than 0.10, preferably less than 0.095 and more preferably less than 0.090. For a more detailed discussion of the term "ionic strength", see Remington's Pharmeceutical Sciences, 17^{th} ed., published by Philadelphia College of Pharmacy and Science (1985).

The contact lens is preferably an oxygen permeable hard lens that canies an ionic charge or has the potential of having an ionic charge.

The charge of the lens surface is anionic, while that of the polymer in the ophthalmic solution is a cellulosic polymer of cationic charge. The cellulosic polymer should be compatible with the eye, should be non-irritating and yet should form a hydrogel that is electrostatically bound to the surface of the contact lens.

Preferably the lens coating is formed by merely immersing the lens in a solution which consists essentially of a cationic cellulose polymer dissolved in a water solution or a water solution containing soluble organic components comprising from 0.001 to 10% by weight of the solution. The polymer can be any cationic cellulose polymer compatible with the eye and which does not cause eye irritation yet which forms a hydrogel and which is electrostatically bound to the surface of the contact lens.

It is a feature of this invention that thin coatings of from 20 to 2,500 Angsnoms are formed, which coatings not only increase the compatibility of contact lens with the eye but also add a cushioning effect between the lens and the eye. Such coatings can reduce problems of punctate staining and further enhance the ability of the contact lens to be worn in the eye for periods up to 24 hours or more.

Depending on the concentration of ionic sites on the lens surface and the concentration of oppositely charged ionic polymer with which the surface is reacted, either wetting, soaking, or lubricating solutions can be prepared to provide optimal wearer comfortability. In addition, if cleaning agents are mixed with the ionic polymer solution, mucus, dirt and other unwanted deposits can be removed from the resulting polyelectrolyte complex surface.

### DESCRIPTION OF PREFERRED EMBODIMENTS

Soft and hard synthetic polymer contact lens materials are normally prepared from neutral monomers and/or polymers. In this invention both soft and hard contact lens materials are prepared in such a manner that ionic sites are present on the lens surface, such sites can be reacted with a lens solution containing an oppositely charged, hydrophilic polymer. If the surface of the lens is considered polyanionic, the surface can then be reacted with a hydrophilic polycation with the resulting formation of a hydrophilic polyelectrolyte complex. Polyelectrolyte complexes have an equal amount of cations and anions, each obtained from a different source. In addition, these overall electrically neutral complexes exist as ionically crosslinked hydrogels that are effective in retaining water of hydration. In this invention, a surface coating of polyelectrolyte complex is achieved on a lens surface. A soft contact lens prepared entirely from a polyelectrolyte complex is known but would not have the desired properties of lenses preferred in accordance with this invention.

The lens solution forms a coating that acts to lubricate the lens. It can be used by immersing the lens in it, spraying it on a lens or other methods. It can also be used in the form of eye drops to be applied to the eye and act as an artificial tear to lubricate the lens and eye.

Polyelectrolyte complexes, although highly hydrophilic, are water-insoluble and can be dissolved with some difficulty usually by a ternary solvent system incorporating water, a water-soluble organic compound, and a low molecular weight electrolyte. This solubility behavior implies that in the present invention the polyelectrolyte complex treated surface is very difficult to dissolve and separate from the lens surface by the aqueous fluids of the eye, although this surface coating conceivable could be eroded by mechanical action in the eye during wear. Should dissipation of the polyelectrolyte complex from the lens surface occur, it can readily be replaced by re-treatment of the lens with the appropriate oppositely charged polyion solution.

The polyelectrolyte complex on the lens surface can be achieved by several means. If an anionic surface is desired as required according to the claims this can be accomplished by incorporation into the lens formulation of any monomer or monomers from the acrylate or methacrylate salt group, a vinyl sulfonate salt, an allyl or methallyl sulfonate or sulfate salt, a styrene sulfonate salt, an acryloyloxyethyl or methacryloyloxyethyl sulfate salt, a substituted acrylamido or methacrylamido sulfonate salt or from related phosphonate, phosphate and phosphite salts of polymerizable monomers. Alternatively, a potentially anionic surface can be generated for subsequent treatment with a polycation followed by elimination of a low molecular weight acid (such as hydrogen chloride) or by subsequent treatment with a neutral basic polymer resulting in an acid-base neutralization reaction. Such anionic monomers include compounds such as acrylic and methacrylic acid, vinylsulfonic acid, allyl or methallyl sulfonic or sulfuric acid, styrene sulfonic acid, an acrylamido or methacrylamido sulfonic acid, or a polymerizable phosphonic or phosphoric acid.

If a cationic surface is desired, this is accomplished by incorporation into the lens formulation of any quaternary or protonated monomer or monomers from the acrylate or methacrylate salt group, a vinylpyridinium salt, a vinylimidazolium salt, a vinylimidazolinium salt, a vinylthiazolium salt, a vinylbenzylammonium salt, a diallyldialkylammonium salt, or a related alkylated or protonated polymerizable sulfonium or phosphonium salt. Alternatively, a potentially cationic surface can be generated for subsequent treatment with a polyacid resulting in an acid-base neutralization reaction. Such potentially cationic monomers include compounds such as a dialkylaminoethyl acrylate or methacrylate, a vinylpyridine, a vinylimidazole, a vinylbenzyl amine, a vinyl alkyl ether or sulfide, or a polymerizable vinyl phosphine.

It is also possible to generate an ionic charge on the lens surface by chemically or electrically modifying a neutral monomeric repeat unit to one that is charged. For example, an anionic surface can be obtained by treating a polyester material, such as poly(methyl methacrylate), with an aqueous base, such as sodium hydroxide, to yield sodium methacrylate units on the lens surface. Alternatively, a polyester material can be hydrolyzed with an acid to yield methacrylic acid units on the lens surface which function as potential anionic sites. Similarly, a cationic surface can be obtained by alkylating or protonating a nucleophilic amine, sulfide or phosphine units on the lens surface.

A form of polyelectrolyte complex can be obtained through reaction of a polyacid surface with a solution of a hydrogen-bonding polymer such as poly(N-vinylpyrrolidone) or polyethylene oxide. Conversely, a hydrogen bonding surface can be treated with a polyacid. Such a polymer-polymer complex occurs through the hydrogen bonding of the polyacid with the acid-receptive groups of poly(N-vinylpyrrolidone) or poly(ethylene oxide).

Many hard and soft contact lens materials presently disclosed are electrically neutral polymers or copolymers. Such materials can be modified to include ionic surface groups. One general method for all types of lenses would include treatment of the surface with high energy irradiation in the presence of air to generate ionic surface groups, see A. Chaprio, Radiation Chemistry of Polymeric Systems, Vol. XV, Interscience, New York, 1962, and F. A. Makhlis, Radiation Physics and Chemistry of Polymers, Wiley and Sons, New York, 1975.

Another method would include modification of polymeric lens formulations through incorporation of ionic (or potentially ionic) monomers. Poly(methyl methacrylate) is amenable to such modification. Examples of this approach include the copolymerization of either acrylic acid, methacrylic acid or dimethylaminoethyl methacrylate to provide a poly(methyl methacrylate) lens with ionic groups on the surface.

Another example would include the modification of oxygen permeable lens formulations such as those in U.S. Pat. No. 3,808,178. These formulations are copolymers of methyl methacrylate with a siloxanyl alkyl ester of methacrylic acid and can be modified through the addition of either acrylic acid, methacrylic acid or dimethylaminoethyl methacrylate.

The invention is especially advantageous for silicone rigid-gas-permeable lenses. Both rigid-gas-permeable ("RGP") materials and hydrogels are well-known classes of materials.

RGP materials typically comprise a hydrophobic crosslinked polymer system containing less than 5 wt. % water. RGP materials useful in accordance with the present invention include those materials taught in US Patent No. 4,826,936 to Ellis; 4,463,149 to Ellis; 4,604,479 to Ellis; 4,686,267 to Ellis et al.; 4,826,936 to Ellis; 4,996,275 to Ellis et al.; 5,032,658 to Baron et al.; 5,070,215 to Bambury et al.; 5,177,165 to Valint et al.; 5,177,168 to Baron et al.; 5,219,965 to Valint et al.; 5,336,797 to McGee and Valint; 5,358,995 to Lai et al.; 5,364,918 to Valint et al.; 5,610,252 to Bambury et al.; 5,708,094 to Lai et al; and 5,981,669 to Valint et al. US Patent 5,346,976 to Ellis et al. teaches a preferred method of making an RGP material.

Hydrogels comprise hydrated, crosslinked polymeric systems containing water in an equilibrium state. Silicone hydrogels generally have a water content greater than about five weight percent and more commonly between about ten to about eighty weight percent. Such materials are usually prepared by polymerizing a mixture containing at least one silicone-containing monomer and at least one hydrophilic monomer. Either the silicone-containing monomer or the hydrophilic monomer may function as a crosslinking agent (a crosslinker being defined as a monomer having multiple polymerizable functionalities) or a separate crosslinker may be employed. Applicable silicone-containing monomeric units for use in the formation of silicone hydrogels are well known in the art and numerous examples are provided in U.S. Patent Nos. 4,136,250; 4,153,641; 4,740,533; 5,034,461; 5,070,215; 5,260,000; 5,310,779; and 5,358,995. The present invention is especially advantageous for application to contact lenses, either silicone hydrogels or silicone rigid-gas-permeable materials. The invention is especially advantageous for silicone hydrogel continuous-wear lenses. Hydrogels are a well-known class of materials, which comprise hydrated, crosslinked polymeric systems containing water in an equilibrium state.

Examples of applicable silicon-containing monomeric units include bulky polysiloxanylalkyl (meth)acrylic monomers. An example of bulky polysiloxanylalkyl (meth)acrylic monomers is represented by the following Formula I: 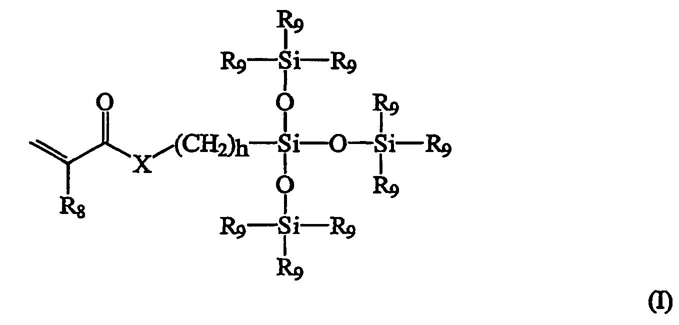
wherein:
X denotes -O- or -NR-;
each R₈ independently denotes hydrogen or methyl;
each R₉ independently denotes a lower alkyl radical, phenyl radical or a group represented by
wherein each R₁₉ independently denotes a lower alkyl or phenyl radical; and
h is 1 to 10.

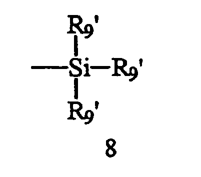

Some preferred bulky monomers are methacryloxypropyl tris(trimethylsiloxy)silane or tris(trimethylsiloxy)silylpropyl methacrylate, sometimes referred to as TRIS and tris(trimethylsiloxy)silylpropyl vinyl carbamate, sometimes referred to as TRIS-VC.

Such bulky monomers may be copolymerized with a silicone macromonomer, which is a poly(organosiloxane) capped with an unsaturated group at two or more ends of the molecule. U.S. Patent No. 4,153,641 to Deichert et al. discloses, for example, various unsaturated groups, including acryloxy or methacryloxy.

Another class of representative silicone-containing monomers includes silicone-containing vinyl carbonate or vinyl carbamate monomers such as: 1,3-bis[4-vinyloxycarbonyloxy)but-1-yl]tetramethyldisiloxane; 3-(trimethylsilyl)propyl vinyl carbonate; 3-(vinyloxycarbonylthio)propyl[tris(trimethylsiloxy)silane]; 3-[tris(trimethylsiloxy)silyl] propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; and trimethylsilylmethyl vinyl carbonate.

Another class of silicon-containing monomers includes polyurethane-polysiloxane macromonomers (also sometimes referred to as prepolymers), which may have hard-soft-hard blocks like traditional urethane elastomers. Examples of silicone urethanes are disclosed in a variety or publications, including Lai, Yu-Chin, "The Role of Bulky Polysiloxanylalkyl Methacryates in Polyurethane-Polysiloxane Hydrogels," Journal of Applied Polymer Science, Vol. 60, 1193-1199 (1996). PCT Published Application No. WO 96/31792 and US Patents No. 5,451,617 and 5,451,651 disclose examples of such monomers. Further examples of silicone urethane monomers are represented by Formulae II and III:

(II) E(*D*A*D*G)ₐ*D*A*D*E';

or

(III) E(*D*G*D*A)ₐ*D*G*D*E';

wherein:
D denotes an alkyl diradical, an alkyl cycloalkyl diradical, a cycloalkyl diradical, an aryl diradical or an alkylaryl diradical having 6 to 30 carbon atoms;
G denotes an alkyl diradical, a cycloalkyl diradical, an alkyl cycloalkyl diradical, an aryl diradical or an alkylaryl diradical having 1 to 40 carbon atoms and which may contain ether, thio or amine linkages in the main chain;
* denotes a urethane or ureido linkage;
a is at least 1;
A denotes a divalent polymeric radical of Formula IV: 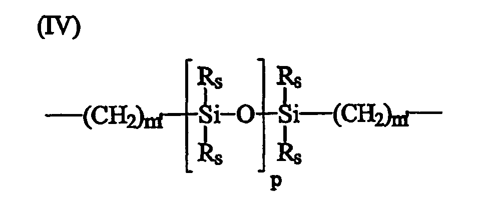
wherein:
each Rs independently denotes an alkyl or fluoro-substituted alkyl group having 1 to 10 carbon atoms which may contain ether linkages between carbon atoms;
m' is at least 1; and
p is a number that provides a moiety weight of 400 to 10,000;
each of E and E' independently denotes a polymerizable unsaturated organic radical represented by Formula 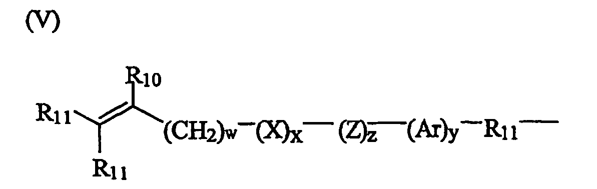
wherein:
R₁₀ is hydrogen or methyl;
R₁₁ is hydrogen, an alkyl radical having 1 to 6 carbon atoms, or a -CO-Y-R₁₃ radical wherein Y is -O-, -S- or NH-;
R₁₂ is a divalent alkylene radical having 1 to 10 carbon atoms;
R₁₃ is a alkyl radical having 1 to 12 carbon atoms;
X denotes -CO- or -OCO-;
Z denotes -O- or -NH-;
Ar denotes an aromatic radical having 6 to 30 carbon atoms;
w is 0 to 6; x is 0 or 1; y is 0 or 1; and z is 0 or 1.

A preferred silicone-containing urethane monomer is represented by Formula (VI): 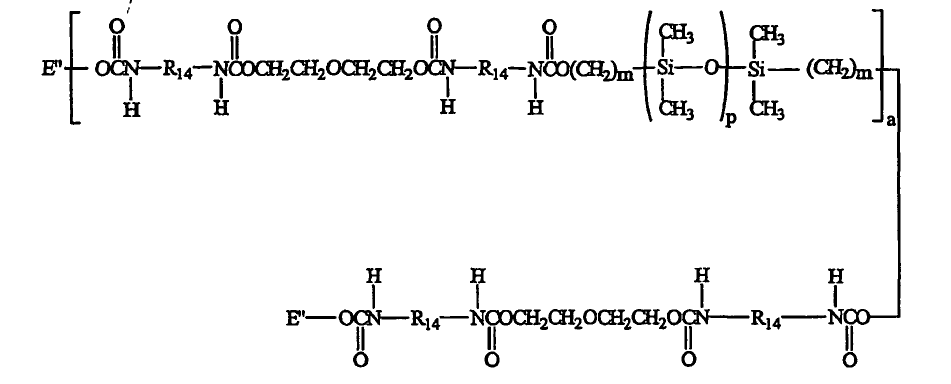
wherein m is at least 1 and is preferably 3 or 4, a is at least 1 and preferably is 1, p is a number which provides a moiety weight of 400 to 10,000 and is preferably at least 30, R₁₄ is a diradical of a diisocyanate after removal of the isocyanate group, such as the diradical of isophorone diisocyanate, and each E" is a group represented by:

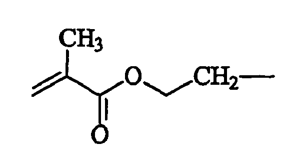

Another class of representative silicone-containing monomers includes fluorinated monomers. Such monomers have been used in the formation of fluorosilicone hydrogels to reduce the accumulation of deposits on contact lenses made therefrom, as described in U.S. Patent Nos. 4,954,587, 5,079,319 and 5,010,141. The use of silicone-containing monomers having certain fluorinated side groups, i.e. -(CF₂)-H, have been found to improve compatibility between the hydrophilic and silicone-containing monomeric units, as described in U.S. Patent Nos. 5,387,662 and 5,321,108.

In one embodiment a silicone hydrogel material comprises (in bulk, that is, in the monomer mixture that is copolymerized) 5 to 50 percent, preferably 10 to 25, by weight of one or more silicone macromonomers, 5 to 75 percent, preferably 30 to 60 percent, by weight of one or more polysiloxanylalkyl (meth)acrylic monomers, and 10 to 50 percent, preferably 20 to 40 percent, by weight of a hydrophilic monomer. Examples of hydrophilic monomers include, but are not limited to, ethylenically unsaturated lactam-containing monomers such as N-vinylpyrrolidinone, methacrylic and acrylic acids; acrylic substituted alcohols, such as 2-hydroxyethyl methacrylate and 2-hydroxyethyl acrylate and acrylamides, such as methacrylamide and N,N-dimethylacrylamide, vinyl carbonate or vinyl carbamate monomers such as disclosed in U.S. Patent Nos. 5,070,215, and oxazolinone monomers such as disclosed in U.S. Patent No. 4,910,277. Other hydrophilic monomers will be apparent to one skilled in the art.

The synthetic resin lens preferably has a total ionic charge of from 0.001% to 10%. Thus from 0.001% to 10% of the surface area is charged and the charge density often is about 5%.

The lens solutions used in accordance with this invention are preferably in all cases USP sterile. The solutions are preferably aqueous isotonic solutions. Preferably they are water solutions containing ingredients common to lens solutions such as buffers, preservatives and viscosity modifiers and which carry from 0.001 to 10% by weight of a water soluble cellulosic polymers containing N,N-dimethylamino-2-hydroxylpropyl groups (either protonated of quaternized). Cationic cellulosic polymers are commercially available or can be prepared by methods known in the art. As an example, quaternary nitrogen-containing ethoxylated glucosides can be prepared by reacting hydroxyethyl cellulose with a trimethylammonium-substituted epoxide. Various preferred cationic cellulosic polymers are commercially available, for example water-soluble polymers available under the CTFA (Cosmetic, Toiletry, and Fragrance Association) designation Polyquaternium-10. Such polymers are commercially available under the tradename UCARE® Polymer from Amerchol Corp., Edison, NJ, USA. These polymers contain quaternized N,N-dimethylamino groups along the cellulosic polymer chain.

The cationic cellulosic component may be employed in the compositions at 0.01 to ten (10) weight percent of the composition, preferably at 0.05 to about five (5) weight percent, with 0.1 to one (1) weight percent being especially preferred. Suitable cationic cellulosic materials have the following formula: 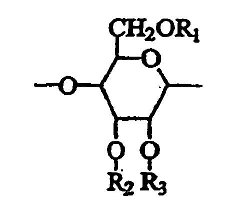 Wherein R₁ R₂ and R₃ are selected from H, derivatives of C₁-C₂₀ carboxylic acid, C₁-C₂₀ alkyl groups, C₁ to C₃ monohydric and dihydric slkanols, hydroxyethyl groups, hydroxypropyl groups, ethylene oxide groups, propylene oxide groups, phenyl groups, "Z" groups and combinations thereof. At least one of R₁, R₂ ,and R₃ is a Z group.

The nature of the "Z" groups is: 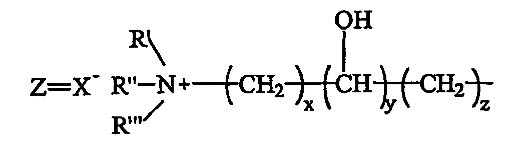
where:
R', R" and R''' can be H, CH₃, C₂H₅, CH₂CH₂OH and 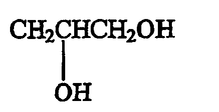
x=0-5, y=0-4, and z=0-5
X⁻ = Cl⁻, Br⁻, I⁻, HSO₄⁻, CH₃SO₄⁻, H₂PO₄⁻, NO₃⁻

The ophthalmic solutions used in accordance with this invention contain a cationic cellulosic material such as, but not limited to:
a cellulose polymer containing N,N-dimethylaminoethyl groups either protonated or quaternized
a cellulose polymer containing 1-(N,N-dimethylamino-2-hydroxylpropyl) groups either protonated or quaternized

Other additives to the soaking lens solutions of this invention include conventional lens solution cleaning and soaking solutions additives. Preservatives such as benzylalkonium chloride, mercurials, trichlorban and chlorobutanol can be used. Wetting agents or viscosity modifiers such as polyvinyl alcohol, hydroxypropyl methylcellulose hydroxy ethyl cellulose, polyvinyl pyrrolidine, polyethylene oxide and methyl cellulose can be used. Lubricating agents such as the wetting agents above but in known higher concentrations can be used. Soaking and cleaning agents such as neutral detergents including sodium dodecyl sulfate and neutral surfactants based on nonyl phenol can be used. Buffers include boric acid, sodium borate, phosphoric acid, disodium phosphate, sodium bicarbonate. Other conventional buffers, biocides and viscosity modifiers may also be used. The solutions may comprise one or more nonionic polymeric of non-polymeric demulcents e.g glycerin, propylene glycol, povidone, poly(vinyl alcohol) and combinations thereof. The additives are used in a wide range of concentrations as known in the art. Preferably the pH of the solutions are as near to body pH as possible and always in the range of pH 6-8, particularly if the solution is to be used only to form the coating of this invention and is to be put directly in the eye when wearing the lens as an artificial tear. Otherwise the pH can vary widely. If used to soak a lens and if the solution is not of a physiological pH the lens can be washed and adjusted in pH before put in the eye. the solutions are aqueous While it is preferred to merely soak the lens in the solution at room temperature, the solution can also be sprayed, dropped, or rubbed on the lens surface. The solutions are rendered sterile by methods common in the art, boiling, autoclaving, gamma irradiation or by membrane filtration.

In all cases it is preferred to form a coating of no more than 2,500A over the lens surface which acts as a hydrogel. The hydrogel formed by the polyelectrolyte complex is an ionically crosslinked polymer that absorbs large amounts of water and at least 10% of its own weight of water. The lenses tend to be non-irritating to the eye and can be worn for long periods of time.

Specific examples of this invention are given below but are not meant in any way to limit this invention.

In the following examples, blanks of a commercial fluorosilicone rigid gas permeable contact lens material (Boston ES® available from Polymer Technology Corporation of Wilmington, MA) were formulated without wetting agents. These blanks were cut into wafers and both sides were polished to an optical finish. The wafers were then soaked in deionized water overnight, and subsequently treated with various solutions as described below. After each treatment with a solution, dynamic contact measurements were taken using Cahn Instrument DCA 315. The results are provided below. The abbreviations used in the Tables below have the following meanings: Adv. = Advancing contact angles in degree; and Rec. = Receding contact angle in degrees.

### Example 1

This example illustrates the ability of a cationic cellulosic polymer to increase the wettability of the surface of a contact-lens material. The following three solutions were prepared by adding a sufficient amount of the indicated constituent to the distilled water in order to achieve the final percentage indicated: (1) 0.1% Polymer JR; (2) 0.1% Mucin (Type 1 from bovine submaxillary glands); and (3) 0.1% Polymer JR and 0.9% sodium chloride a comparative solution. The above-described wafers were sequentially dipped within the solutions indicated in the Table 1. After being treated with each solution, contact angle measurements were taken, the results of which are also provided in the Table 1.

**Table 1**

| **Experimental Condition** | **Adv.** | **Rec.** | **Hysteresis** |
|---|---|---|---|
| **Study One** | | | |
| Baseline in Water (4 cycles) | **97** | **33** | **64** |
| Into 0.1% Polymer JR in Water (7 cycles) | **31** | **23** | **9** |
| 1^{st} Desorption in Water (4 cycles) | **40** | **14** | **26** |
| 2^{nd} Desorption in Water (4 cycles) | **76** | **20** | **56** |

| **Study Two** | | | |
|---|---|---|---|
| Baseline in Water (4 cycles) | **97** | **29** | **68** |
| Into 0.1% Mucin in Water (7 cycles) | **32** | **27** | **5** |
| 1^{st} Desorption in Water (4 cycles) | **61** | **15** | **47** |
| 2^{nd} Desorption in Water (4 cycles) | **77** | **13** | **63** |

| **Study Three** | | | |
|---|---|---|---|
| Baseline in Water (4 cycles) | **98** | **32** | **66** |
| Into 0.1% Polymer JR in Water (7 cycles) | **37** | **25** | **12** |
| Into 0.1% Mucin in Water (2 cycles) | **34** | **29** | **5** |
| 1^{st} Desorption in Water (4 cycles) | **43** | **24** | **19** |
| 2^{nd} Desorption in Water (4 cycles) | **50** | **14** | **36** |

| **Study Four** (comparative) | | | |
|---|---|---|---|
| Baseline in Water (4 cycles) | **98** | **30** | **68** |
| Into 0.1% Polymer JR in 0.9% NaCl (7 cycles) | **26** | **16** | **10** |
| Into 0.1 % Mucin in Water (2 cycles) | **45** | **31** | **15** |
| 1^{st} Desorption in Water (4 cycles) | **64** | **14** | **50** |
| 2^{nd} Desorption in Water (4 cycles) | **71** | **12** | **59** |

Lens treatment with either Polymer JR or Mucin dramatically lowered both the advancing and receding contact angles of the treated wafers. However, the adsorbed polyelectrolyte on the wafer surface is mostly removed during the first and second desorption processes (Studies One and Two). Surprisingly, the durability of the polyelectrolyte layer coating the lens surface is greatly enhanced if the wafers treated with Polymer JR are further exposed to Mucin which is abundant in the human tear (Study Three). This durability became absent when Polymer JR solution contained 0.9% sodium chloride (Study Four), suggesting the involvement of ionic strength in the complex formation.

### Example 2

This example further illustrates the impact of ionic strength on the ability of a cationic cellulosic polymer to coat the surface of a contact-lens material. The three solutions tested contained 0.1% Polymer JR. They were prepared in the following three base medium solutions: (1) the distilled water; (2) the borate buffer containing 1% boric acid and 0.11% sodium borate; and (3) the isotonic phosphate buffer containing 0.28% disodium phosphate, 0.055% monosodium phosphate and 0.73% sodium chloride a comparative solution. The ionic strength increases from solution from the solution (1) to the solution (3). The above-described wafers were sequentially dipped within the solutions indicated in the Table 2. After being treated with each solution, contact angle measurements were taken, the results of which are also provided in the Table 2.

**Table 2**

| **Experimental Condition** | **Adv.** | **Rec.** | **Hysteresis** |
|---|---|---|---|
| **Study One** | | | |
| Baseline in Water (4 cycles) | **103** | **29** | **74** |
| Into 0.1% Polymer JR in Water (7 cycles) | **43** | **23** | **20** |
| 1^{st} Desorption in Water (4 cycles) | **49** | **23** | **26** |
| 2^{nd} Desorption in Water (4 cycles) | **57** | **23** | **34** |

| **Study Two** | | | |
|---|---|---|---|
| Baseline in Water (4 cycles) | **102** | **28** | **74** |
| Into 0.1% Polymer JR in Borate Buffer (7 cycles) | **48** | **25** | **24** |
| 1^{st} Desorption in Water (4 cycles) | **46** | **22** | **24** |
| 2^{nd} Desorption in Water (4 cycles) | **64** | **22** | **42** |

| **Study Three** (comparative) | | | |
|---|---|---|---|
| Baseline in Water (4 cycles) | **102** | **28** | **74** |
| Into 0.1% Polymer JR in Isotonic Phosphate Buffer (7 cycles) | **46** | **27** | **19** |
| 1^{st} Desorption in Water (4 cycles) | **50** | **24** | **26** |
| 2^{nd} Desorption in Water (4 cycles) | **76** | **23** | **53** |

As indicated in Table 2, above, the durability of the polyelectrolyte coating is dependent on the ionic strength of the treatment solution, with lower ionic strengths associated with more durable coatings.

## Claims

1. A method of wetting a contact lens having a surface comprising anionic sites which comprises administering an aqueous solution comprising 0.001 to 10.0 percent by weight of a cationic cellulosic polymer **characterised in that** the solution has an ionic strength of no more than 0.10.

2. A method as claimed in Claim 1 in which the ionic strength is no more than 0.095.

3. A method as claimed in Claim 2 in which the ionic strength is no more than 0.090.

4. A method as claimed in any preceding claim in which the aqueous solution further comprises at least one tonicity agent which is present in an amount of 0.01 to 10.0 percent by weight.

5. A method as claimed in any preceding claim in which the aqueous solution comprises an effective amount of a buffering agent to maintain a pH of from 6 to 8.

6. A method as claimed in any preceding claim in which the aqueous solution further comprises one or more non-ionic polymeric or non-polymeric demulcents.

7. A method as claimed in Claim 6 in which the demulcent is selected from glycerin, propylene glycol and combinations thereof.

8. A method as claimed in Claim 6 in which the demulcent is selected from non-ionic cellulosic polymers, povidone, poly(vinyl alcohol) and combinations thereof.

9. A method as claimed in Claim 6 in which the demulcent is povidone.

10. A method as claimed in any preceding claim in which the contact lens is on an eye and the method comprises instilling in the eye drops of said aqueous solution.

## Patentansprüche

1. Verfahren zum Benetzen einer Kontaktlinse mit einer Oberfläche, die anionische Stellen umfasst, welches das Verabreichen einer wässrigen Lösung umfasst, die 0,001 bis 10,0 Gew.-% eines kationischen Cellulosepolymers umfasst, **dadurch gekennzeichnet, dass** die Lösung eine lonenstärke von nicht mehr als 0.10 aufweist.

2. Verfahren wie In Anspruch 1 beansprucht, in welchem die Ionenstärke nicht mehr als 0.095 beträgt.

3. Verfahren wie in Anspruch 2 beansprucht, in welchem die Ionenstärke nicht mehr als 0.090 beträgt.

4. Verfahren wie in einem der vorangehenden Ansprüche beansprucht, in welchem die wässrige Lösung außerdem wenigstens ein Tonizitätsmittel umfasst, weiches In einer Menge von 0,01 bis 10,0 Gew.-% vorhanden ist.

5. Verfahren wie in einem der vorangehenden Ansprüche beansprucht, in welchem die wässrige Lösung eine wirksame Menge einer Puffersubstanz zum Aufrechterhalten eines pH-Wertes von 6 bis 8 umfasst.

6. Verfahren wie in einem der vorangehenden Ansprüche beansprucht, in welchem die wässrige Lösung außerdem ein oder mehrere nichtionische polymere oder nichtpolymere Milderungsmittel umfasst.

7. Verfahren wie in Anspruch 6 beansprucht, in welchem das Milderungsmittel ausgewählt ist aus Glycerin, Propylenglycol und Kombinationen davon.

8. Verfahren wie in Anspruch 6 beansprucht, in welchem das Milderungsmittel ausgewählt ist aus nichtionischen Cellulosepolymeren, Povidon, Poly(vinylalkohol) und Kombinationen davon.

9. Verfahren wie in Anspruch 6 beansprucht, in welchem das Milderungsmittel Povidon ist.

10. Verfahren wie in einem der vorangehenden Ansprüche beansprucht, in welchem die Kontaktlinse sich auf einem Auge befindet und das Verfahren das Einträufeln von Tropfen der wässrigen Lösung in das Auge umfasst.

## Revendications

1. Procédé de mouillage d'une lentille de contact présentant une surface comportant des sites anioniques, qui comprend une administration d'une solution aqueuse comprenant 0,001 à 10,0 % en poids d'un polymère cellulosique cationique, **caractérisé en ce que** la solution présente un force ionique qui n'est pas supérieure à 0,10.

2. Procédé suivant la revendication 1, dans lequel la force ionique n'est pas supérieure à 0,095.

3. Procédé suivant la revendication 2, dans lequel la force ionique n'est pas supérieure à 0,090.

4. Procédé suivant l'une quelconque des revendications précédentes, dans lequel la solution aqueuse comprend en outre au moins un agent de tonicité qui est présent en une quantité de 0,01 à 10,0 % en poids.

5. Procédé suivant l'une quelconque des revendications précédentes, dans lequel la solution aqueuse comprend une quantité efficace d'un agent de tamponnement pour maintenir un pH de 6 à 8.

6. Procédé suivant l'une quelconque des revendications précédentes, dans lequel la solution aqueuse comprend en outre un ou plusieurs agents adoucissants polymères ou non polymères non ioniques.

7. Procédé suivant la revendication 6, dans lequel l'agent adoucissant est choisi parmi de la glycérine, du propylène glycol et leurs combinaisons.

8. Procédé suivant la revendication 6, dans lequel l'agent adoucissant est choisi parmi des polymères cellulosiques non ioniques, de la povidone, du poly(alcool vinylique) et leurs combinaisons.

9. Procédé suivant la revendication 6, dans lequel l'agent adoucissant est de la povidone.

10. Procédé suivant l'une quelconque des revendications précédentes, dans lequel la lentille de contact est sur un oeil et le procédé comprend une instillation dans l'oeil de gouttes de ladite solution aqueuse.
